# EUROPÄISCHE PATENTSCHRIFT

(11) **EP 2 588 015 B1**
(45) Veröffentlichungstag und Bekanntmachung des Hinweises auf die Patenterteilung: **16.11.2016**
(21) Anmeldenummer: 11730209.1
(22) Anmeldetag: 24.06.2011
(51) Int. Cl.: A61B 17/80, A61B 17/86, A61B 17/00

(54) **FIXATIONSSYSTEM FÜR KNOCHEN**
FIXATION SYSTEM FOR BONES
SYSTÈME DE FIXATION POUR OS

(30) Priorität: 30.06.2010 DE 102010025702
(43) Veröffentlichungstag der Anmeldung: 08.05.2013
(60) Teilanmeldung: 16173279.7
(73) Patentinhaber: AAP Implantate AG, 12099 Berlin (DE)
(72) Erfinder: BATSCH, Thomas, 10367 Berlin (DE); PAULIN, Thomas, 14552 Wilhelmshorst OT Michendorf (DE); FISCHER, Hans-Joachim, 12277 Berlin (DE); ALEMU, Bruke, Seyoum, 14195 Berlin (DE)
(74) Vertreter: Blumbach Zinngrebe
(86) Internationale Anmeldenummer: PCT/EP2011/003112
(87) Internationale Veröffentlichungsnummer: WO 2012/000627

(56) Entgegenhaltungen:
- EP-A1- 1 649 819
- EP-A1- 2 016 918
- EP-A2- 1 949 866
- WO-A1-97/09000
- WO-A2-2004/084701
- WO-A2-2007/014192
- FR-A1- 2 880 929

## Beschreibung

### Gebiet der Erfindung

Die Erfindung bezieht sich auf ein Fixationssytem für Knochen mit Knochenschrauben und einer Knochenplatte, die mehrere in Richtung der Plattenlängsachse angeordnete Löcher für die Aufnahme von Knochenschrauben aufweist.

### Hintergrund der Erfindung

Bei einer bekannten Knochenplatte (EP 0760632 B1) sind die Löcher zur Oberseite der Knochenplatte hin rundum sphärisch ausgebildet, um eine Knochenschraube mit kugeliger Unterseite des Kopfes in unterschiedlichen Winkelstellungen abstützen zu können. An der Plattenunterseite benachbart zum Knochen weisen die Löcher einen Bereich kleineren Durchmessers mit partiellem Gewinde auf, um auch eine Knochenschraube mit zylindrischem Gewindekopf aufnehmen zu können, die senkrecht zur Plattenebene eingesenkt werden soll.

Bei einer weiteren Knochenplatte (EP 1158915 B1 und EP 1158916 B1) sind Langlöcher mit einem Innengewinde, das sich an einem Ende des Langlochs von der Oberseite bis zur Unterseite der Knochenplatte erstreckt und einen Umfangs- oder Zentriwinkel im Bereich von 190-280°aufweist, vorhanden. Das Innengewinde nimmt die gesamte Tiefe des Langloches ein, verjüngt sich gegen die Unterseite der Knochenplatte hin konisch und weist einen Konuswinkel im Bereich von 5 bis 20°auf.

Bei einer weiteren bekannten Knochenplatte (EP 1255498 B1) sind Langlöcher in der Knochenplatte vorgesehen, die oval, elipsenförmig oder auch rechteckig ausgebildet sein können oder eine Kombination solcher Formen beinhalten; lediglich kreisrunde Löcher sind von dieser Definition des Langlochs explizit ausgeschlossen worden. Das Langloch ist mit einem kreisförmigen Loch kombiniert und dieses ist mit einer dreidimensionalen Strukturierung versehen, welche in Form eines Innengewindes oder einer peripheren Lamelle oder Lippe vorliegt. Dargestellt ist ein konisches Innengewinde, das sich von der Oberseite bis zur Unterseite der Knochenplatte erstreckt und einen Umfangs- oder Zentriwinkel im Bereich von 190 bis 280° aufweist.

Aus DE 19858889 A1 ist ein Fixationssystem für Knochen mit einer Knochenplatte bekannt, welche Langlöcher als Durchgangslöcher besitzt, die nahe ihrer dem Knochen benachbarten Unterseite Vorsprünge aufweist, welche sich im unteren Teil des Langloches parallel zur Plattenebene erstrecken. Zur Oberseite der Knochenplatte hin gibt es Sitzflächen für Kugelköpfe der Knochenschrauben. Die Sitzflächen sind als nach innen geneigte Konusflächen oder Kugelflächen ausgebildet. Um mit den Vorsprüngen in der Knochenplatte zusammen zu arbeiten, weist die Knochenschraube unterhalb des Kugelkopfes ein kurzes Stück Gewinde auf, das in der Lage ist, die nachgiebigen Vorsprünge in dem Langloch zu deformieren und sich anzuverwandeln. Die Sitzflächen der Knochenplatte und der Knochenschraube machen es möglich, die Knochenschraube in verschiedenen Winkelstellungen bezüglich der Achse der Durchgangslöcher festzulegen.

Die Offenlegungsschrift EP 1 859 752 A1 zeigt ein Fixationssystem, aus dem der Oberbegriff des Anspruchs 1 abgeleitet wird, mit einer Knochenplatte und einer Knochenschraube, welches geeignet ist, mit einer Schraube die Bruchstücke eines Knochens zu komprimieren und gleichzeitig eine winkelstabile Verbindung einzugehen.

Die Knochenplatte weist längliche Lochausbildungen auf, welche teilweise mit einem Gewinde mit mehreren Gewindegängen versehen sind.

Unterhalb des Gewindes ist eine Stützfläche für einen korrespondierenden Teil eines unteren Abschnitts des Kopfes der Knochenschraube angeordnet. Oberhalb der korrespondierenden Stützfläche der Knochenschraube weist diese ein Kopfgewinde auf.

### Allgemeine Erfindungsbeschreibung

Der Erfindung liegt die Aufgabe zugrunde, ein Fixationssytem mit Knochenschrauben und einer Knochenplatte zu schaffen, bei dem verschiedenartige Knochenschrauben - solche mit Konussitzflächen und solche mit Kugelsitzflächen - angewendet werden können, um den unterschiedlichen Anforderungen beim Fixieren eines gebrochenen oder geschädigten Knochens zu genügen. Insbesondere sollen Knochenfragmente beim Fixieren relativ zueinander verschoben werden können.

Das neue Fixationssystem ist aus zwei Arten Knochenschrauben und einer Knochenplatte aufgebaut, wobei mit der ersten Art Knochenschrauben nur Verschraubung senkrecht zur Plattenform der Knochenplatte möglich ist, die zu diesem Zweck eine radiale Rippe und eine konische Sitzfläche aufweist, während mit der zweiten Art Knochenschraube im Schrägwinkel zur Plattenebene gearbeitet werden kann, wobei kein Eingriff der Knochenschraube in die radiale Rippe erfolgt. Die zweite Art der Knochenschraube weist teilsphärische Flächen an der Kopfunterseite auf, jedoch kein Gewinde zum Eingriff in die Knochenplatte.

Im einzelnen weist die Knochenplatte einen vorzugsweise länglichen Plattenkörper aus einem ersten, gewebeverträglichen, steifen Material auf, der eine Oberseite und Unterseite und eine Längsachse bestimmt. Quer zur Plattenebene sind Lochausbildungen vorgesehen, die aus einem ersten, größeren Rundloch und einem zweiten, kleineren Rundloch bestehen, wobei sich die Rundlöcher unter Bildung von Kanten schneiden, zwischen denen ein Durchlass für den Schraubenschaft einer schräg angesetzten Knochenschraube gebildet wird. Um beide Rundlöcher herum ist eine radiale Rippe vorgesehen, die von der Lochwandlung ausgeht und sich in einer Ebene auf die Rundlochmitte hin erstreckt. Die erste Art der Knochenschraube ist mit Gewinde am Schraubkopf versehen und kann sich gegebenenfalls an der Rippe des kleineren Rundloches abstützen und sich mit der Rippe des größeren Rundloches verschrauben, wodurch Deformation und gegenseitige Formanpassung erfolgt.

Im Sinne der Erfindung behält diese Deformation auch nach dem Trennen der Knochenschraube von der Knochenplatte einen bleibenden Anteil, dies bedeutet, dass diese Deformation über den rein elastischen Anteil hinaus auch plastische Deformationen umfasst.

Dieser plastisch deformierte, bleibende Anteil der Deformation kann je nach Material und Ausgestaltung der konstruktiven Abmessungen allein in der Knochenschraube vorliegen, kann allein in der Knochenplatte vorliegen oder kann auch in beiden, sowohl der Knochenschraube als auch der Knochenplatte vorliegen.

Typischerweise liegt der plastische, verbleibende Anteil in jeweils einer Art Furche oder Rille vor, welche mindestens eine Tiefe von 10 µm, bevorzugt von mehr als 100 µm und besonders bevorzugt von mehr als 200 µm aufweist.

Gemäß bevorzugter Ausführungsform weist das erste, größere Rundloch drei Abschnitte auf, nämlich einen oberen, rundkehlförmigen Abschnitt oberhalb der umlaufenden Rippe, einen mittleren, rundkehlförmigen Abschnitt unterhalb der umlaufenden Rippe sowie einen unteren, kegelstumpfförmigen Abschnitt, der sich zur Unterseite des Plattenkörpers hin verjüngt und dessen größter Durchmesser kleiner als der Durchmesser des oberen oder mittleren Abschnitts ist. Das kleinere Rundloch umfasst einen oberen Abschnitt mit einem geneigten Übergangsbereich zur Plattenoberseite hin, ferner einen mittleren Abschnitt unterhalb der Ebene der umlaufenden Rippe mit geneigt-abgerundeter Fläche sowie einen unteren, zylindrischen oder konischen Abschnitt mit einem Durchmesser kleiner als der Durchmesser des oberen oder mittleren Abschnitts.

Bei allen Ausführungsformen lassen sich Knochenschrauben der ersten Art mit einem Kopf verwenden, der in seinem oberen Bereich mit Schraubgewinde und in seinem unteren Bereich mit einer konischen Sitzfläche versehen ist, und zwar in der Weise, dass beim exzentrischen Einschrauben senkrecht zur Plattenebene in einen Knochen eine Verschiebung des Kopfes relativ zu der Lochausbildung stattfindet, dergestalt, dass Knochenbruchstücke beim Fixieren aneinander angenähert werden können. Die neue Knochenplatte ermöglicht aber auch die Anwendung von Knochenschrauben der zweiten Art mit sphärischen Sitzflächen an der Unterseite des Schraubkopfes. Solche Kugelkopfschrauben können im Winkel zu der Knochenplatte eingeschraubt werden, wobei der Schraubenschaft im Knochen verankert wird und die Fixierung durch Anlage der sphärischen Sitzfläche des Kopfes an passender Sitzfläche der Knochenplatte erfolgt.

Weitere Einzelheiten der Erfindung ergeben sich aus der Beschreibung der dargestellten Ausführungsbeispiele und den angefügten Ansprüchen.

### Die Zeichnungen zeigen:

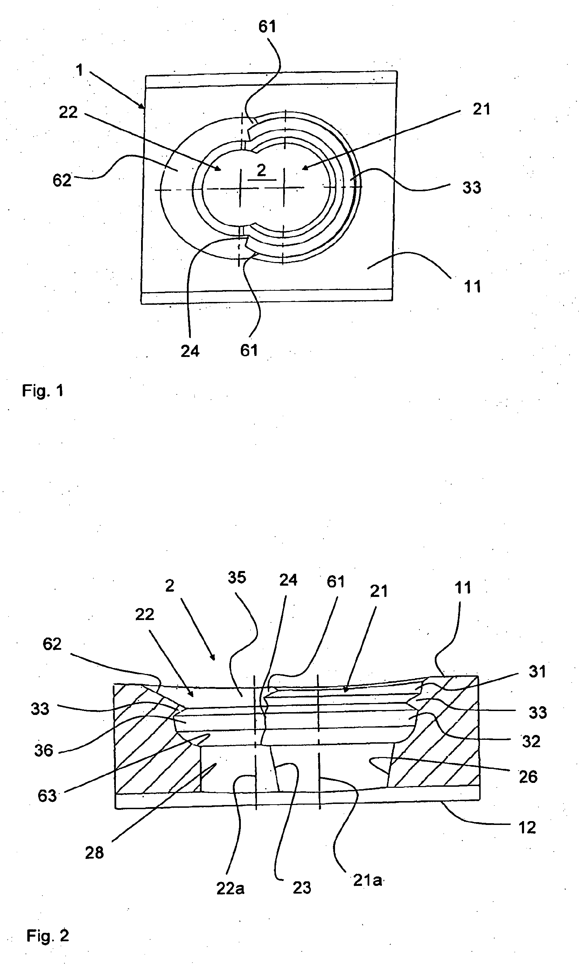

Fig. 1 eine Draufsicht auf eine Lochausbildung in einer Knochenplatte,
Fig. 2 eine Schnittansicht der Lochausbildung,
Fig. 3 eine perspektivische Darstellung der Lochausbildung,
Fig. 4 eine Knochenschraube im Eingriff mit einer Lochausbildung,
Fig. 5 eine zweite Ausführungsform einer Knochenplatte in perspektivischer Darstellung,
Fig. 6 eine Schnittansicht der Lochausbildung,
Fig. 7 eine Seitenansicht der Lochausbildung, und
Fig. 8 eine Knochenschraube im Eingriff mit einer Lochausbildung, zusammen mit einer Ausschnittsvergrößerung.

### Detaillierte Beschreibung bevorzugter Ausführungsformen

Die Figuren zeigen das Stück einer Knochenplatte, die aus einem länglichen Plattenkörper 1 aus einem ersten, gewebevexträglichen, steifen Material besteht und in den eine Reihe von Lochausbildungen angebracht sind, von denen eine Lochausbildung 2 dargestellt ist. Der Plattenkörper 1 kann außer länglich auch oval, rund oder mehreckig ausgebildet oder von seiner Form her der jeweiligen Anwendung angepasst sein.

Als gewebevertragliches Material werden beispielsweise Metalle und deren Legierungen verstanden, wie diese üblicherweise zur Herstellung von Implantaten verwendet werden. Bevorzugte Metalle umfassen Titan in jeder Form, vorzugsweise auch dessen Legierungen TiAl6V4 und TiCp.. Auch Stähle, wie beispielsweise Implantatestahl, beispielsweise die Legierung 1.4441 sind vorteilhaft verwendbar.

Eine weitere Materialklasse umfasst hierfür auch resorbierbare Materialien, wie beispielsweise Magnesium oder PLA. PLA ist ein biokompatibler und resorbierbarer Kunststoff aus chemisch aneinander gebundenen Milchsäuremolekülen, der wie auch andere resorbierbare Kunststoffe verwendet werden kann.

Als steif wird eine Knochenplatte dann angesehen, wenn diese konstruktiv die für ihre vorgesehene Verwendung und Einsatzzweck nötige Steifigkeit zur Verfügung stellt. Dies kann je nach verwendetem gewebeverträglichen Material durch die Dicke und Breite der Platte sichergestellt werden und liegt üblicherweise im Bereich fachmännischen Handelns.

So sind beispielsweise Knochenplatten für kleine und weniger belastete Körperpartien, wie beispielsweise Hand- und Fußknochen dünner und häufig weniger breit als Knochenplatten der größeren und stärker belasteten Partien, wie beispielsweise bei Teilen des Unter- und des Oberschenkels.

Die Knochenplatte weist eine Oberseite 11 und eine Unterseite 12 auf, die gewöhnlich parallel zur Plattenebene verlaufen, wobei die Unterseite 12 dem zu fixierenden Knochen benachbart ist. Die Lochausbildungen 2 sind entlang der Längsachse des länglichen Plattenkörpers 1 aufgereiht und bestehen aus zwei stufig ausgebildeten Rundlöchern, 21 und 22, die sich quer zur Plattenebene erstrecken und deren Achsen 21a, 22a die Längsachse der Knochenplatte schneiden. Der kleinste Durchmesser des ersten Rundlochs 21 ist größer als der kleinste Durchmesser des zweiten Rundlochs 22 und der Abstand der beiden Achsen 21a und 22a voneinander ist kleiner als der kleinste Durchmesser des ersten Rundlochs 21.

Durch die Überschneidung der Löcher 21, 22 ergeben sich Kanten 23, 24, welche die Bereiche der Rundlöcher gegeneinander abgrenzen und einen Durchlass für den relativ dünnen Schraubschaft einer Knochenschraube freilassen, wenn bei der Schrägstellung einer Knochenschraube diese von einem Rundloch in das andere Rundloch hineinreicht. Die Umfangswinkel der Wandlaibungen der Rundlöcher 21, 22 betragen ca. 250° des größeren Loches und ca. 220° des kleineren Loches. Variationen um 10° kleiner und 20° größer sind möglich.

Infolge der Stufigkeit der Rundlöcher 21, 22 kann man eine obere Region 25 und eine untere Region 26 des Rundloches 21 und eine obere Region 27 sowie eine untere Region 28 des Rundlochs 22 unterscheiden.

Die oberen Regionen 25 und 27 sind schüsselförmig ausladend gestaltet, während die unteren Regionen 26 und 28 Mantelflächen mit geraden Mantellinien bilden. Die oberen Regionen 25 und 27 weisen größere Durchmesser gegenüber den unteren Regionen 26 und 28 auf. Die untere Region 26 bildet einen kegelstumpfförmigen Abschnitt, der sich zur Unterseite des Plattenkörpers 1 hin verjüngt. Die untere Region 28 ist zylindrisch ausgebildet, sie kann aber auch kegelstumpfförmig ausgebildet sein.

Während die beiden oberen Regionen 25 und 27 der beiden Rundlöcher 21, 22 insgesamt schüsselförmig gestaltet sind, zieht sich, ausgehend von den Lochwandungen, eine radiale Rippe 33 in einer Ebene um die Lochausbildung 2 herum. Die Rippe 33 ist als umlaufender Steg ausgebildet und weist einen keilförmigen Querschnitt auf, der sich zur Mitte des jeweiligen Rundloches hin verjüngt. In der Draufsicht erscheint die Rippe 33 einer Acht ähnlich zu sein.

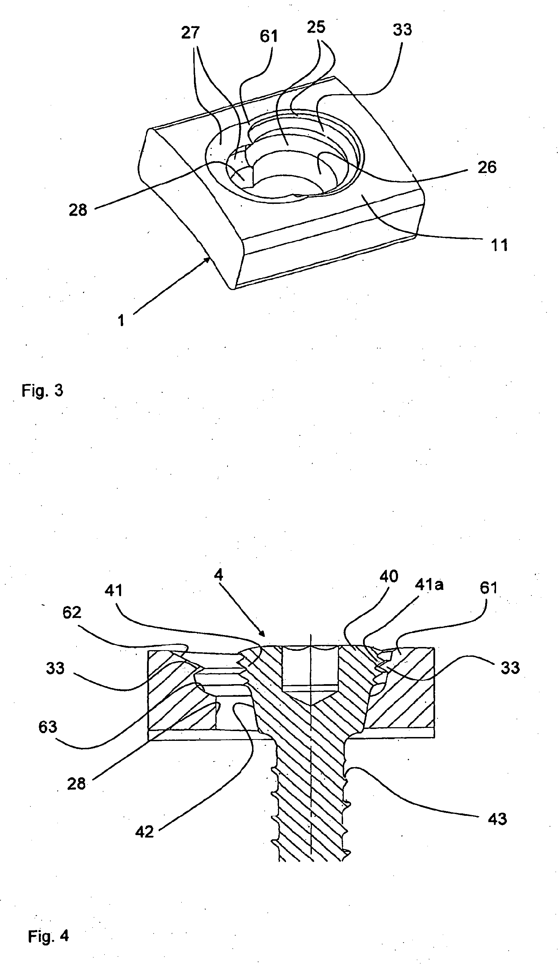

Während die Rippe 33 gleichmäßig herumlaufen kann, wird für den am größeren Rundloch angebrachten Rippenteil ein Grat 61 parallel zur Ebene der Rippe 33 am Außenrand des größeren Rundlochs 21 angeordnet, wodurch sich eine Kante 24 zum zweiten Rundloch 22 hin bildet. Ausgehend von der Längsachse des Plattenkörpers 1 nimmt die Höhe des Grates 61 zum Rand des Plattenkörpers 1 hin zu, wie am besten aus dem Vergleich der Fig. 4 mit Fig. 3 hervorgeht. Zwischen dem Grat 61 und der Rippe 33 wird eine Gangführung gebildet, die den Eingriff des Außengewindes 41 der Kopfschraube 40 begünstigt.

Jedes der beiden Rundlöcher 21, 22 ist in drei Abschnitte unterteilt: Das größeren Rundloch 21 weist oberhalb der Rippe 33 einen oberen, rundkehlförmigen Abschnitt 31 mit, oder auch ohne Grat 61 auf, und unterhalb der Rippe 33 sind ein mittlerer, rundkehlförmiger Abschnitt 36 und ein unterer, kegelstumpfförmiger Abschnitt 26 vorgesehen. Das kleinere Rundloch 22 umfasst einen oberen Abschnitt 35 mit Einführungsschräge 62, einen mittleren Abschnitt 36 mit einer geneigt-abgerundeten Fläche 63 unterhalb der Ebene der Rippe 33, und einen unteren Abschnitt 28, der vorzugsweise zylindrisch ausgebildet ist, aber auch konisch sein kann.

Die Knochenplatte ist zur Zusammenwirkung mit wenigstens zweierlei Arten von Knochenschrauben ausgebildet. Die zweite Art weist einen Knochenschraubenkopf mit teilsphärischer Unterseite auf und kann sich mit der Kopfunterseite an der geneigt-abgerundeten Fläche 63 der Knochenplatte abstützen. Dabei ist auch Schrägstellung der Schraubenachse gegenüber der Plattenebene möglich, und zwar sowohl in Längsrichtung als auch (im geringen Maße) in Querrichtung zur Knochenplatte. Ermöglicht wird dies durch den Abstand der Kanten 23 voneinander, der größer ist als der Durchmesser des Schraubschaftes der Knochenschraube.

Die erste Art von anwendbarer Knochenschraube 4 ist in Fig. 4 dargestellt. Diese Knochenschraube 4 weist einen Schraubkopf 40 mit Inneneingriff und mit Außengewinde 41 am oberen Ende sowie konischer Stützfläche 42 am unteren Ende auf, deren Konusneigung der Konusneigung der unteren Region 26 des größeren Rundloches 21 entspricht. Das Außengewinde 41 kann zylindrisch sein, es wird jedoch konisches Gewinde bevorzugt.

Die Konusneigung der unteren Region 26 des größeren Rundloches 21 sowie die Konusneigung der konischen Stützfläche 42 am unteren Ende des Schraubkopfs 40 der Knochenschraube weist einen Winkel relativ zur Längs- oder Symmetrieachse jeweils der Knochenschraube oder des Rundlochs 21 im Bereich von 3 bis 30° auf. Bevorzugt liegt dieser Konuswinkel in einem Bereich von 6 bis 20° und am bevorzugtesten in einem Bereich von 8 bis 12°. Eine besonderst bevorzugte Bauform hat sich mit einem Konuswinkel von etwa 10° mit hohen Dauerfestigkeitswerten und guter Lösbarkeit der Verbindung zwischen Knochenschraube und Knochenplatte sehr bewährt.

In einem Winkelbereich von etwa 8 bis 12° werden sehr gute Stabilitätswerte gegenüber dem Verkippen der Knochenschraube relativ zur Knochenplatte bei gleichzeitig nur moderater Selbsthemmung bereitgestellt.

An den Schraubkopf 40 schließt sich ein Schraubschaft 43 an, der dazu bestimmt ist, in einem zu fixierenden Knochenelement verankert zu werden. Die umlaufende Rippe 33 erstreckt sich in einer Ebene, vorzugsweise parallel zur Plattenebene, während sich das Außengewinde 41 entlang von Schraubflächen erstreckt, die naturgemäß schräg gegenüber der Ebene der radialen Rippe 33 verlaufen, und zwar auch dann, wenn die Knochenschraube 4 bestimmungsgemäß parallel oder gleichlaufend mit der Achse 21a des Rundloches 21 verschraubt wird. Dabei erfolgt gegenseitige Formanpassung zwischen den Gewindegängen des, Außengewindes 41 und der Rippe 33. In dieser Hinsicht ist der Grat 61 nützlich, da er ein Gegenlager für das Gewinde des Schraubkopfes bietet.

Die gegenseitige Formaripassung setzt eine gewisse Nachgiebigkeit des (ersten) Materials der Knochenplatte wenigstens im Bereich der radialen Rippe 33 und/oder des (zweiten) Materials der Knochenschraube wenigstens im Bereich des Außengewinde 41 voraus. Es wird bevorzugt, das (zweite) Material der Knochenschraube härter als das (erste) Material der Knochenplatte zu machen und auch eine Form mit einem größeren Widerstandsmoment für das Außengewinde 41 gegenüber dem der Rippe 33 zu wählen. Auf diese Weise passt sich die "weichere", in einer Ebene liegende Rippe weitgehend der Eingriffsschraubwindung des Außengewindes 41 an, wenn das Fixationssystem bestimmungsgemäß angewendet wird.
Dennoch liegt es auch im Rahmen der Erfindung, dass mindestens im Bereich der radialen Rippe 33 und des Außengewindes 41 das erste und zweite Material gleiche Härten aufweisen.

Im Zusammenhang mit der dargestellten Knochenplatte kann die Knochenschraube 4 zur gegenseitigen Annährung und zum Zusammenpressen von Knochenfragmenten benutzt werden. Hierzu wird die Knochenschraube 4 mit ihrer Achse parallel zur Achse 22a im Bereich des Rundloches 22 angesetzt. Sobald der untere Rand der konischen Stützfläche 42 die Einführungsschräge 62 des kleineren Rundloches 22 erreicht, erfolgt beim Eindrehen der Schraube eine seitliche Kraft auf den Schraubkopf 40, was zu einer Verschiebung des zu fixierenden Knochenfragments relativ zu der Knochenplatte führt. Wenn demnach ein Knochenfragment mit der Knochenplatte bereits fest verbunden ist, wird dieses Knochenfragment gegen das zu fixierende Knochenfragment geschoben, wie es erwünscht ist.

Es wird darauf hingewiesen, dass wegen des großen Umfangwinkels der Konusfläche der unteren Region 26, die im Bereich von 250 ° bis 290° liegt, bei festsitzendem Schraubkopf 40 eine genügend starke Verbindung zwischen dem Plattenkörper 1 und der Knochenschraube 4 hergestellt wird, da nach gegenseitiger Deformation die umlaufende Rippe 33 und das Außengewinde 41 genügend elastische Spannkraft entwickeln, um die konischen Flächen bei 26 und 42 aufeinandergepresst zu halten.

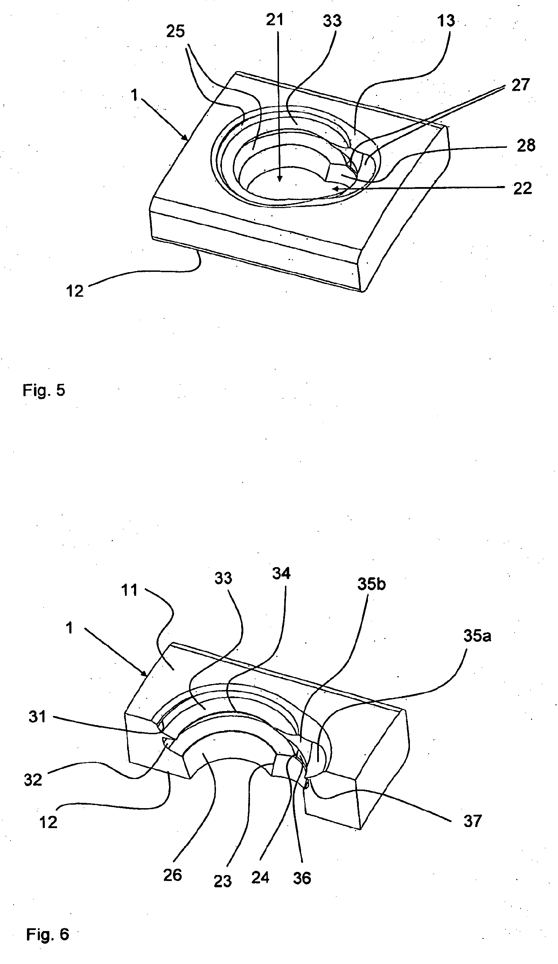

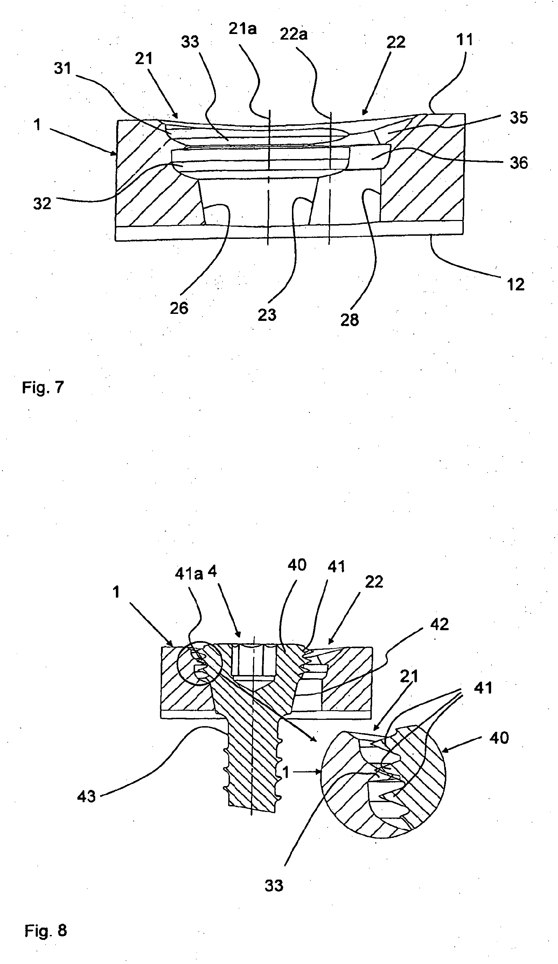

Mit Fig. 5 - 8 ist eine weitere besonders bevorzugte Ausführungsform der Knochenplatte dargestellt, wobei die gleichen Bezugszeichen für sich entsprechende Teile verwendet werden.

Der hauptsächliche Unterschied besteht in der Ausbildung der umlaufenden radialen Rippe 33. Diese ist im Bereich des kleineren Rundlochs 22 teilweise weggeschnitten, um eine geneigt-abgerundete Gleitfläche 35a und eine Übergangsfläche 35b zu schaffen, die zur Führung des Kopfes einer Knochenschraube mit teilsphärischer Unterseite des Kopfes oder zur Führung der Schraube mit konischer Stützfläche 42 dienlich sind. Es wird eine Rastrippe 37 gebildet, die auf volle Größe der Rippe 33 im Bereich des größeren Rundloches 21 ansteigt. Die verbleibende Randkante 34 erstreckt sich um weniger als 180° und erlaubt so die seitliche Einführung von Knochenschrauben vom kleineren Rundloch 22 in das größere Rundloch 21.

Die Rippe 33 hat einen keilförmigen Querschnitt mit einer zur Lochmitte geneigten Oberseite und einer Unterseite parallel zur Plattenebene. Auf diese Weise ist das Widerstandsmoment der Rippe 33 bedeutend kleiner als das Widerstandsmoment des Außengewindes 41 im Eingriff mit der Rippe, die Rippe 33 also nachgiebiger selbst dann, wenn die Materialien von Rippe und Schraubwindung von gleicher Härte sind. Eine geringere Härte der Rippe 33 wird jedoch zu Zwecken der Formanpassung bevorzugt.

Mit der Erfindung wird ein Fixationssystem für Knochen mit Knochenplatte und Knochenschrauben geschaffen, bei dem Knochenschrauben mit Rundkopf im unterschiedlichen Schrägwinkel zur Knochenplatte gesetzt werden können. Ferner ermöglicht das Fixationssytem bei der Anwendung von Knochenschrauben mit Kegelkopf die Relativverschiebung zwischen zu fixierendem Knochenfragment und Knochenplatte, was es dem Operateur ermöglicht, Knochenfragmente bei deren Fixierung gegeneinander zu verschieben.

## Patentansprüche

1. Fixationssystem mit einer Knochenplatte und einer Knochenschraube, um Teile eines gebrochenen oder geschädigten Knochens relativ zueinander zu verschieben und zu fixieren,
wobei die Knochenplatte einen Plattenkörper (1), der eine Plattenebene und eine Längsachse bestimmt, umfasst und Lochausbildungen (2) quer zur Plattenebene aufweist,
wobei die Knochenschraube (4) einen Schraubkopf (40) mit im oberen Bereich des Kopfes angebrachtem Außengewinde (41) und mit im unteren Bereich angebrachter konischer Sitzfläche (42), sowie mit einem Schraubenschaft (43) zum Einschrauben in den zu fixierenden Knochen umfasst,
**dadurch gekennzeichnet, dass** die Knochenplatte aus einem ersten, steifen Material und die Knochenschraube aus einem zweiten, steifen Material besteht,
dass die Lochausbildungen aus einem ersten Rundloch (21) und einem zweiten Rundloch (22) bestehen, welche stufig ausgebildet sind und sich unter Bildung von Kanten (23,24) schneiden und eine Schraubeneinführungsseite sowie eine Schraubenaustrittsseite besitzen, wobei das erste Rundloch (21) an der Schraubeneinführungsseite eine obere Region (25) und an der Schraubenaustrittsseite eine als konische Sitzfläche ausgebildete untere Region (26) aufweist, wobei sich die Kanten (24) durch die obere Region (25) und die Kanten (23) durch die untere Region (26) erstrecken, und
wenigstens das erste Rundloch (21) eine radiale Rippe (33) aufweist, die sich in einer Ebene in der oberen Region (25) zur Rundlochmitte hin erstreckt, und
wobei beim Einschrauben der Knochenschraube (4) in den Knochen sich die konische Sitzfläche (42) der Knochenschraube (4) auf der konischen Sitzfläche (26) der Knochenplatte abstützt, so dass bei der Zusammenarbeit von Außengewinde (41) und radialer Rippe (33) eine Deformation erfolgt, die eine gegenseitige Anpassung der Rippe (33) und eines Eingriffsgewindeganges (41a) des Außengewindes der Knochenschraube (4) zur Folge hat.

2. Fixationssystem nach Anspruch 1,
wobei mindestens im Bereich der radialen Rippe (33) und des Außengewindes (41) das erste und zweite Material unterschiedliche Härten aufweisen.

3. Fixationssystem nach Anspruch 1,
wobei mindestens im Bereich der radialen Rippe (33) und des Außengewindes (41) das erste und zweite Material gleiche Härten aufweisen.

4. Fixationssystem nach Anspruch 1, 2 oder 3,
wobei die Form und Härte der radialen Rippe (33) eine größere Nachgiebigkeit der Rippe (33) gegenüber dem Eingriffsgewindegang (41a) der Knochenschraube (4) zur Folge hat.

5. Fixationssystem nach Ansprüchen 1 bis 4,
wobei die radiale Rippe (33) von den Rundlochwandungen beider Rundlöcher (21,22) ausgeht und um diese vollständig herumführt.

6. Fixationssytem nach Anspruch 5,
wobei die umflaufende, radiale Rippe (33) im Bereich des zweiten Rundlochs (22) reduziert ist, um eine geneigt-abgerundete Gleitfläche (35a) und eine Übergangsfläche (35b) zu bilden

7. Fixationssytem nach Ansprüchen 5 oder 6,
wobei die umlaufende Rippe (33) im zweiten Rundloch (22) zur Oberseite (11) der Knochenplatte hin mit Einführungsschräge (62) verläuft.

8. Fixationssystem nach einem der Ansprüche 1 bis 7,
wobei das erste Rundloch (21) größer als das zweite Rundloch (22) ist und
seine obere Region (25) Schüsselform aufweist und mit einem zugehörigen Abschnitt der radialen Rippe (33) versehen ist, und die
untere als konische Sitzfläche ausgebildetete Region (26) einen kleineren Durchmesser gegenüber dem Durchmesser der oberen Region aufweist.

9. Fixationssystem nach Anspruch 8,
wobei das zweite, kleinere Rundloch (22) eine obere Region (27) in Schüsselform und mit einem zugehörigen Abschnitt der radialen Rippe (33) umfasst, sowie eine untere Region (28) mit einer zylindrischen oder konischen Fläche, deren Durchmesser kleiner ist als der Durchmesser der oberen Region, aufweist.

10. Fixatinossystem nach Anspruch 8 oder 9,
wobei die obere Region (25) des ersten, größeren Rundlochs (21) durch die Rippe (33) in einen oberen, rundkehlförmigen Abschnitt (31) oberhalb der Rippe (33) und in einen mittleren, rundkehlförmigen Abschnitt (36) unterhalb der Rippe (33) unterteilt ist und die untere Region (26) des größeren Rundlochs (21) als ein unterer, kegelstumpfförmiger Abschnitt ausgebildet ist, der sich zur Unterseite (12) des Plattenkörpers (1) hin verjüngt und dessen größter Durchmesser kleiner als der Durchmesser des oberen oder mittleren Abschnitts (31, 32) ist.

11. Fixationssystem nach Anspruch 10,
wobei die obere Region (27) des zweiten, kleineren Rundloches (22) einen oberen Abschnitt (35) mit Einführungsschräge (62) aufweist und einen mittleren Abschnitt (36) mit einer geneigt-abgerundeter Fläche (63) unterhalb der Ebene der Rippe (33) besitzt.

12. Fixationssytem nach einem der Ansprüche 1 bis 11,
wobei die Rippe (33) einen keilförmigen Querschnitt aufweist.

## Claims

1. A fixation system comprising a bone plate and a bone screw for displacing relative to each other and fixing parts of a fractured or damaged bone;
wherein the bone plate comprises a plate body (1) defining a plate plane and a longitudinal axis and having hole formations (2) transversely to the plate plane;
wherein the bone screw (4) has a screw head (40) with an outer thread (41) formed in the upper region of the head and a conical bearing surface (42) formed in the lower region of the head, and a screw shaft (43) for being screwed into the bone to be fixed;
**characterized in that** the bone plate is made of a first rigid material and the bone screw is made of a second rigid material;
that the hole formations comprise a first round hole (21) and a second round hole (22) of stepped configuration which intersect to define edges (23, 24) and have a screw insertion side and a screw exit side, wherein the first round hole (21) has an upper region (25) on the screw insertion side and a lower region (26) shaped as a conical bearing surface on the screw exit side, with the edges (24) extending across the upper region (25) and the edges (23) across the lower region (26), and wherein at least the first round hole (21) has a radial rib (33) which extends in a plane in the upper region (25) towards the centre of the round hole; and wherein upon screwing of the bone screw (4) into the bone the conical bearing surface (42) of the bone screw (4) is supported on the conical support surface (26) of the bone plate, so that the external thread (41) and the radial rib (33) cooperate to cause a deformation resulting in a mutual adaptation of the rib (33) and an engaging thread turn (41a) of the external thread of the bone screw (4).

2. The fixation system according to claim 1, wherein at least in the region of the radial rib (33) and of the external thread (41) the first and second materials have different hardnesses.

3. The fixation system according to claim 1, wherein at least in the region of the radial rib (33) and of the external thread (41) the first and second materials have the same hardness.

4. The fixation system according to claim 1, 2, or 3, wherein the shape and hardness of the radial rib (33) causes a greater resilience of the rib (33) compared to the engaging thread turn (41a) of the bone screw (4).

5. The fixation system according to claims 1 to 4, wherein the radial rib (33) extends from the round hole walls of both round holes (21, 22) extending completely around the holes.

6. The fixation system according to claim 5, wherein the circumferential radial rib (33) is reduced in the region of the second round hole (22) so as to form an inclined rounded sliding surface (35a) and a transition surface (35b).

7. The fixation system according to claims 5 or 6, wherein in the second round hole (22) the circumferential rib (33) extends with an introductory slope (62) towards the upper side (11) of the bone plate.

8. The fixation system according to any of claims 1 to 7, wherein the first round hole (21) is greater than the second round hole (22) and is bowl-shaped in its upper region (25) and is provided with an associated portion of the radial rib (33); and
wherein the lower region (26) in the form of a conical support surface has a smaller diameter than the diameter of the upper region.

9. The fixation system according to claim 8, wherein the second, smaller round hole (22) has a bowl-shaped upper region (27) with an associated portion of the radial rib (33) and a lower region (28) with a cylindrical or conical surface having a diameter that is smaller than the diameter of the upper region.

10. The fixation system according to claim 8 or 9, wherein the upper region (25) of the first, larger round hole (21) is divided by the rib (33) into an upper, round neck-shaped portion (31) above the rib (33) and a central, round neck-shaped portion (36) below the rib (33), and the lower region (26) of the larger round hole (21) is formed as a lower, truncated cone-shaped portion which tapers towards the lower surface (12) of the plate body (1) and has a maximum diameter which is smaller than the diameter of the upper or central portions (31, 32).

11. The fixation system according to claim 10, wherein the upper region (27) of the second, smaller round hole (22) comprises an upper portion (35) having an introductory slope (62) and a central portion (36) having an inclined rounded surface (63) below the plane of the rib (33).

12. The fixation system according to any of claims 1 to 11, wherein the rib (33) has a wedge-shaped cross section.

## Revendications

1. Système de fixation comprenant une plaque osseuse et une vis à os, destiné à déplacer les unes par rapport aux autres des parties d'un os brisé ou endommagé et de les fixer,
la plaque osseuse comprenant un corps de plaque (1), qui détermine un plan de plaque et un axe longitudinal, et des configurations de trous (2) transversaux au plan de plaque,
la vis à os (4) comprenant une tête (40) de vis pourvue d'un filetage extérieur (41) agencé dans la zone supérieure de la tête et d'une embase conique (42) agencée dans la zone inférieure, ainsi que d'une tige (43) de vis destinée à être vissée dans l'os à fixer,
**caractérisé en ce que** la plaque osseuse est constituée d'un premier matériau rigide et la vis osseuse est constituée d'un deuxième matériau rigide,
**en ce que** les configurations de trous sont constituées d'un premier trou rond (21) et d'un deuxième trou rond (22), lesquels sont étagés et se coupent, ce qui entraîne la formation d'arêtes (23, 24), et possèdent un côté d'introduction de vis ainsi qu'un côté de sortie de vis, le premier trou rond (21) comprenant, sur le côté d'introduction de vis, une région supérieure (25) et, sur le côté de sortie de vis, une région inférieure (26) réalisée sous la forme d'une embase conique, les arêtes (24) s'étendant à travers la région supérieure (25) et les arêtes (23) s'étendant à travers la région inférieure (26), et
au moins le premier trou rond (21) comprenant une nervure radiale (33), qui s'étend dans un plan dans la région supérieure (25) en direction du centre du trou, et
lorsque la vis à os (4) est vissée dans l'os, l'embase conique (42) de la vis à os (4) s'appuie sur l'embase conique (26) de la plaque osseuse, de sorte que lorsque le filetage extérieur (41) et la nervure radiale (33) coopèrent, une déformation se produit, qui entraîne une adaptation mutuelle de la nervure (33) et d'un pas de filetage de contact (41a) du filetage extérieur de la vis à os (4).

2. Système de fixation selon la revendication 1,
dans lequel le premier et le deuxième matériau présentent des duretés différentes au moins dans la zone de la nervure radiale (33) et du filetage extérieur (41).

3. Système de fixation selon la revendication 1,
dans lequel le premier et le deuxième matériau présentent des duretés identiques au moins dans la zone de la nervure radiale (33) et du filetage extérieur (41).

4. Système de fixation selon la revendication 1, 2, ou 3,
dans lequel la forme et la dureté de la nervure radiale (33) entraînent une flexibilité accrue de la nervure (33) par rapport au pas de filetage de contact (41a) de la vis à os (4).

5. Système de fixation selon les revendications 1 à 4,
dans lequel la nervure radiale (33) fait saillie des parois des trous ronds des deux trous ronds (21, 22) et passe intégralement autour de ces derniers.

6. Système de fixation selon la revendication 5,
dans lequel la nervure radiale (33) circulaire est réduite dans la zone du deuxième trou rond (22), afin de former une surface de glissement inclinée-arrondie (35a) et une surface de transition (35b).

7. Système de fixation selon l'une quelconque des revendications 5 et 6,
dans lequel la nervure circulaire (33) s'étend dans le deuxième trou rond (22) en direction de la face supérieure (11) de la plaque osseuse au moyen d'une pente d'introduction (62).

8. Système de fixation selon l'une quelconque des revendications 1 à 7,
dans lequel le premier trou rond (21) est plus grand que le deuxième trou rond (22) et
sa région supérieure (25) présente une forme de cuvette et est pourvue d'une section associée de la nervure radiale (33), et la région (26) réalisée sous la forme d'une embase conique présente un diamètre inférieur au diamètre de la région supérieure.

9. Système de fixation selon la revendication 8,
dans lequel le deuxième trou rond (22) plus petit comporte une région supérieure (27) sous forme de cuvette et pourvue d'une section associée de la nervure radiale (33), ainsi qu'une région inférieure (28) présentant une surface cylindrique ou conique, dont le diamètre est inférieur au diamètre de la région supérieure.

10. Système de fixation selon la revendication 8 ou 9,
dans lequel la région supérieure (25) du premier trou (21) plus grand est divisée par la nervure (33) en une section supérieure (31) en forme de gorge ronde au-dessus de la nervure (33) et en une section centrale (36) en forme de gorge ronde au-dessous de la nervure (33) et la région inférieure (26) du trou rond (21) plus grand est réalisée sous la forme d'une section inférieure tronconique, qui se rétrécit en direction du côté inférieur (12) du corps de plaque (1) et dont le plus grand diamètre est inférieur au diamètre de la section supérieure ou centrale (31, 32).

11. Système de fixation selon la revendication 10,
dans lequel la région supérieure (27) du deuxième trou rond (22) plus petit comprend une section supérieure (35) présentant une pente d'introduction (62) et une section centrale (36) présentant une surface inclinée-arrondie (63) au-dessous du plan de la nervure (33).

12. Système de fixation selon l'une quelconque des revendications 1 à 11,
dans lequel la nervure (33) présente une section transversale cunéiforme.
